# EUROPÄISCHE PATENTSCHRIFT

(11) **EP 3 216 491 B1**
(45) Veröffentlichungstag und Bekanntmachung des Hinweises auf die Patenterteilung: **23.01.2019**
(21) Anmeldenummer: 17159192.8
(22) Anmeldetag: 03.03.2017
(51) Int. Cl.: A61N 1/372, H05K 1/16

(54) **EMPFÄNGER**
RECEIVER
RÉCEPTEUR

(30) Priorität: 07.03.2016 DE 102016104115
(43) Veröffentlichungstag der Anmeldung: 13.09.2017
(73) Patentinhaber: WITTENSTEIN SE, 97999 Igersheim (DE)
(72) Erfinder: Matthes, Michael, 97999 Igersheim (DE); Hammel, Sebastian, 97959 Assamstadt (DE)
(74) Vertreter: Zimmermann & Partner Patentanwälte mbB

(56) Entgegenhaltungen:
- WO-A1-02/094370
- WO-A1-2009/108233
- US-A1- 2004 082 977
- US-A1- 2011 270 349
- US-A1- 2013 199 027

## Beschreibung

### Gebiet der Erfindung

Die Erfindung betrifft einen Empfänger, insbesondere einen implantierbaren Empfänger zur Übertragung von Energie zu einem Implantat, ein implantierbares System und ein Verfahren zur Herstellung eines Empfängers.

### Stand der Technik

Aus dem Stand der Technik sind implantierbare Empfänger für Implantate bekannt, welche über eine Spule zum Empfangen von Energie oder Signalen verfügen. Bei bekannten Empfängern wird eine Kupferlackdraht-Spule auf die Leiterplatte aufgelötet und auf die Leiterplatte aufgeklebt. Typischerweise sind aufgrund der Größe und der Anzahl der elektrischen Bauteile zwei Leiterplatten nötig, welche manuell aufeinander aufgesetzt werden und mit Stiften verbunden werden müssen. Dies ist mit vielen Montageschritten verbunden, wobei diese Schritte auch fehleranfällig sind.

Die US 8,521,303 beschreibt eine beispielhafte implantierbare Spulenanordnung aus dem Stand der Technik. Die dort gezeigte Planarspule ist in einer Polymermatrix integriert und kann eine Vielzahl von Spulenschichten umfassen. Allerdings ist die gezeigte Anordnung mit Spule vergleichsweise groß.

Weitere implantierbare Spulenanordnungen sind aus WO 2009/108233, US 2013/199027, sowie WO 02/094370 bekannt.

### Offenbarung der Erfindung

Aufgabe der Erfindung ist es, einen gegenüber dem Stand der Technik verbesserten Empfänger und ein verbessertes Verfahren zur Herstellung eines Empfängers anzugeben. Der Empfänger sollte insbesondere kompakter oder kostengünstiger herzustellen sein.

Die Aufgabe wird z. B. gelöst durch einen Empfänger gemäß Anspruch 1 oder durch ein Verfahren gemäß dem nebengeordneten Anspruch 9. Weiterbildungen des Verfahrens oder der Vorrichtung sind in den abhängigen Ansprüchen angegeben.

Ein erster Aspekt betrifft einen Empfänger, insbesondere implantierbaren Empfänger zur Übertragung von Energie zu einem Implantat, mit einer mehrlagigen Platine, welche eine Mehrzahl von elektrisch leitenden Lagen umfasst, wobei die Platine einen äußeren Spulenbereich und einen von dem Spulenbereich umfangenen mehrlagigen Innenbereich umfasst, einer Spule, welche zumindest teilweise in den Lagen der Platine im Spulenbereich integral aufgenommen ist, wobei innerhalb dieses Innenbereichs die Anzahl der Lagen der Platine geringer ist als in dem Spulenbereich.

Ein weiterer Aspekt betrifft ein implantierbares System mit einem Empfänger in einer der hierin beschriebenen typischen Ausführungsformen und mit einem elektromechanischen Implantat.

Ein weiterer Aspekt betrifft ein Verfahren zum Herstellen eines Empfängers, insbesondere implantierbarer Empfänger zur Übertragung von Energie zu einem Implantat, welcher eine Platine umfasst, mit: Herstellen einer mehrlagigen Grundmembran der Platine mit einer Mehrzahl von Lagen, Aufbau von weiteren Lagen auf einer Oberseite und/oder einer Unterseite der Grundmembran, wobei zumindest in einem Teil der weiteren Lagen im Spulenbereich Windungen der Spule integriert sind, und Schaffen einer oberen Kavität oder einer unteren Kavität aus den weiteren Lagen im Innenbereich innerhalb des Spulenbereichs durch Abtragen, bspw. Ausfräsen oder Ausschleifen der weiteren Lagen im Innenbereich.

Empfänger von Ausführungsformen sind implantierbar, beispielsweise subkutan oder in der Nähe oder an einem Knochen.

Typischerweise ist pro leitender Lage eine oder mehrere Isolierschichten vorgesehen. Beispielsweise können leitende Lagen mit Isolierschichten im Wechsel den Aufbau der Platine bilden. Die jeweils unterste oder oberste Lage kann frei liegen oder mit einer weiteren Isolierschicht bedeckt sein.

Durch die größere Anzahl der Lagen im Spulenbereich wird typischerweise eine obere Kavität ausgebildet, welche durch ein oberes Bord begrenzt wird. Das obere Bord wird typischerweise zumindest teilweise durch die weiteren Lagen im Spulenbereich gebildet, welche oberhalb einer Grundmembran ausgebildet sind. Die Grundmembran umfasst typischerweise den Innenbereich und den Außenbereich. Im Innenbereich ist bei typischen Ausführungsformen nur die Grundmembran der Platine vorhanden, wohingegen im Spulenbereich weitere Lagen ober- oder auch zusätzlich unterhalb der Grundmembran ausgebildet sein können. Die weiteren Lagen auf der Unterseite im Spulenbereich bilden typischerweise ein unteres Bord.

Typischerweise sind Windungen der Spule in dem Spulenbereich ausgebildet. Dies ermöglicht eine platzsparende und geschützte Anordnung der Windungen in den Lagen im Spulenbereich. Bei typischen Ausführungsformen sind zwischen 3 und 15 Windungen pro Lage, insbesondere zwischen 5 und 10 oder insbesondere exakt 7 Windungen pro Lage vorgesehen. Bei typischen Ausführungsformen umfasst der Spulenbereich drei oder mehr Lagen umfasst, beispielsweise mindestens zwei Lagen der Grundmembran oder mindestens zwei weitere Lagen im Spulenbereich. Typischerweise sind in der Grundmembran oder im Innenbereich mindestens 3 oder mindestens 4 Lagen vorhanden. Typischerweise sind in der Grundmembran oder im Innenbereich höchstens 6 Lagen oder höchstens 10 Lagen angeordnet. Bei typischen Ausführungsformen sind im Spulenbereich mindestens 8 weitere Lagen oder mindestens 12 weitere Lagen angeordnet. Bei typischen Ausführungsformen sind im Spulenbereich höchstens 16 weitere Lagen oder höchstens 25 weitere Lagen vorhanden.

Typische Empfänger sind für ein elektromechanisches Implantat geeignet. Typische elektromechanische Implantate sind insbesondere Distraktionseinrichtungen, die bspw. für eine Behandlung langer Röhrenknochen oder von Skoliose geeignet sind. Typischerweise ist der Empfänger dazu eingerichtet, Energie für eine elektrische Antriebsmaschine eines aktiven Implantats bereitzustellen, beispielsweise eine Leistung von mindestens 0,1 Watt oder mindestens 0,5 Watt. Typische Ausführungsformen sind zur Energieversorgung von aktiven Implantaten geeignet, wobei unter aktiv typischerweise verstanden wird, dass das Implantat eine Bewegung ausführen kann oder einen Antriebsmotor umfasst.

Die weiteren Lagen werden bei typischen Verfahren zur Herstellung eines Empfängers sowohl im Innenbereich als auch Spulenbereich aufgebracht. Eine typische Lage der Lagen der Grundmembran oder auch der weiteren aufgebrachten Lagen umfassen Leiterbahnen oder typischerweise eine leitende Schicht pro Lage.

Bei typischen Verfahren wird mit dem Ausfräsen des Innenbereichs im Spulenbereich ein oberes Bord und im Innenbereich die obere Kavität ausgebildet; wobei außerdem zusätzlich im Spulenbereich ein unteres Bord und im Innenbereich auf der Unterseite die untere Kavität ausgebildet werden kann.

Typischerweise umfasst der Innenbereich höchstens oder weniger als halb so viele Lagen wie der Spulenbereich. Auf diese Weise wird eine obere Kavität und eventuell auch eine untere Kavität zur Aufnahme von elektronischen Bauelementen geschaffen.

Bei typischen Ausführungsformen bildet der Spulenbereich auf einer Oberseite der Platine ein oberes Bord um den Innenbereich aus, wobei in dem Innenbereich auf der Oberseite der Platine elektronische Bauelemente angeordnet sind. Typischerweise ist eine maximale Höhe der elektronischen Bauelemente auf der Oberseite höchstens doppelt typischerweise höchstens 1,5-fach oder höchstens 1,2-fach oder höchstens 1,1-fach so hoch wie die Stufe zwischen Spulenbereich und Innenbereich auf der Oberseite. Diese Stufe entspricht der Höhe zwischen der Oberseite der Grundmembran und dem oberen Rand des oberen Bordes. Typischerweise gelten die Grenzen für die Höhen analog für elektronische Bauelemente, welche in einer bei manchen Ausführungsformen vorhandenen unteren Kavität angeordnet sind gegenüber dem Rand des unteren Bordes. Durch die Höhengrenzen wird eine geschützte Aufnahme der Bauelemente erreicht und ein flaches oder gleichmäßiges Profil des Empfängers geschaffen.

Bei typischen Empfängern bildet auf einer Unterseite der Platine der Spulenbereich oder die weiteren Lagen des Spulenbereichs ein unteres Bord aus, welches auf der Unterseite eine untere Kavität im Innenbereich umfängt.

Bei Ausführungsformen von Empfängern können elektronische Bauteile von der Grundmembran oder den weiteren Lagen des Spulenbereichs umschlossen sein oder im Innenbereich in die Lagen insbesondere der Grundmembran integriert sein.

Typischerweise ist die Platine einstückig ausgeführt. Bei alternativen Ausführungsformen sind zumindest zwei Platinen vorgesehen, wobei eine extra für die Spule vorgesehen sein kann. Es können zusammenklappbare, verklebte Starrflexkonstruktionen verwendet werden. Durch diese Anordnung entsteht im Produktionsprozess der Platine eine Grundmembran, bei der auf der Unterseite weitere Lagen aufgebaut wurden. Bei typischen Ausführungsformen wird zunächst nur eine untere Kavität oder keine Kavität geschaffen. Die Oberseite der Platine ist somit leichter zu bestücken. Die Platine besitzt zusätzlich zu Innenbereich und Spulenbereich noch einen flexiblen Bereich. Über diesen flexiblen Bereich kann ein weiterer Spulenbereich nach der Bestückung der elektronischen Komponenten über die Platine geklappt und verklebt werden. Durch diese Ausführungsform ist eine höhere Induktivität der Spule oder auch eine höhere Strombelastung möglich. Als Material für den flexiblen Bereich kann Polyimid zum Einsatz kommen.

Typische Empfänger umfassen eine funkwellenlose Rückmeldeeinrichtung, welche dazu eingerichtet ist, eine Rückmeldung über einen Betriebszustand eines an den Empfänger angeschlossenen Implantats zu erzeugen. Typischerweise ist die Rückmeldeeinrichtung in der unteren Kavität oder in der oberen Kavität angeordnet. Typischerweise kann diese auch von der Grundmembran oder dem Spulenbereich umschlossen sein oder im Innenbereich in die Lagen insbesondere der Grundmembran integriert sein.

Ein möglicher Betriebszustand kann die Funktionsfähigkeit eines an den Empfänger angeschlossenen Implantats oder eine Bewegungsrichtung eines Antriebs eines an den Empfänger angeschlossenen Implantats sein.

Typische Empfänger umfassen einen Schalter zum Verändern des Betriebszustandes des an den Empfänger angeschlossenen Implantats. Typische Schalter umfassen: Reed-Kontakt, Fotodiode oder elektromechanischer Druckschalter. Typische Schalter arbeiten frei von elektromagnetischer Strahlung und sind damit unabhängig von einer Funkwellen-Übertragung, zu welcher typischerweise die Spule genutzt werden kann.

Bei typischen Ausführungsformen wird die obere Kavität oder die untere Kavität mit einem Harz-Härter-Gemisch ausgegossen. Dabei kann das obere bzw. das untere Bord im Spulenbereich als Gussform dienen. Typischerweise werden beide Kavitäten ausgegossen.

Typische Empfänger werden mit einem biokompatiblen Material umspritzt, bspw. Silikon oder Epoxidharz. Möglich wäre bei Ausführungsformen auch ein Glasgehäuse oder ein Keramikgehäuse in der Form, dass Halbschalen verklebt oder miteinander verschweißt werden.

Um das biokompatible Material möglichst nicht zu beschädigen werden bei Ausführungsformen von Empfängern die Kanten der Leiterplatte mit einem Radius versehen. Auf diese Weise können scharfe Kanten vermieden werden und eine Implantierbarkeit begünstigt werden.

Typischerweise ist der Innenbereich der Grundmembran auf zumindest einer der Oberseite und der Unterseite während des Aufbauens der weiteren Lagen durch eine Schutzschicht bedeckt. Typische Schutzschichten umfassen beispielsweise Teflon oder bestehen aus Teflon. Die Schutzschicht kann in einem im Innenbereich ausgeschnittenem Prepreg bzw. einer im Innenbereich ausgeschnittenen Isolationsschicht angeordnet sein.

Zur Herstellung der Platine können bekannte Herstellverfahren aus der Leiterplattentechnik zur Herstellung von Multilayer Circuit Boards eingesetzt werden, die eine Integration der Spule in die Platine ermöglichen oder auch eine selektive Erzeugung von Höhenniveaus ermöglichen.

Nach der Fertigung der Platine und ggf. einem Ausfräsen der Kavität oder der Kavitäten wird bei typischen Ausführungsformen Lotpaste in der Kavität appliziert. Dies kann z.B. durch Jetprinten oder Dispensen durchgeführt werden.

Typische Verfahren umfassen außerdem ein Bestücken des Innenbereichs an der Oberseite mit elektronischen Bauelementen, ein Ausgießen der oberen Kavität mit einem Harz-Härter-Gemisch oder ein Umspritzen des Empfängers mit einem biokompatiblen Material.

Typischerweise sind im Spulenbereich mindestens 10 Lagen, typischerweise mindestens 20 Lager oder mindestens 24 Lagen vorgesehen. Typische Ausführungsformen umfassen höchstens 100 Lagen, typischerweise höchstens 50 Lagen. Typische Spulen umfassen mindestens 50 Windungen, mindestens 100 Windungen oder mindestens 160 Windungen. Typischerweise umfasst die Spule höchstens 500 Windungen oder höchstens 200 Windungen.

Typische Vorteile von Ausführungsformen sind ein kompakter Aufbau bei teilweise deutliche erhöhter Funktionalität oder optimiertem Wirkungsgrad gegenüber bekannten Empfängern von aktiven Implantaten. Typische Empfänger weisen eine Höhe von weniger als 5 mm oder von weniger als 4 mm auf. Typischerweise beträgt die Stufe des oberen Bordes mindestens 1 mm oder mindestens 2 mm. Typische Höhen des unteren Bordes sind maximal 1 mm oder maximal 0,5 mm. Typische Durchmesser betragen mindestens 15 mm oder mindestens 20 mm oder höchstens 30 mm oder höchstens 50 mm. Typischerweise liegt die Dicke der Grundmembran bei mindestens 5% oder mindestens 7% oder höchstens 20% oder höchstens 15% der gesamten Höhe des Empfängers.

Bei typischen Empfänger-Ausführungsformen können mehr Funktionen integriert werden. Ein Verlöten der Spule kann vermieden werden, bspw. kann die Gefahr einer Verpolung reduziert werden.

Typische Ausführungsformen sind robuster, da weniger Einzelteile verwendet werden und sowohl die Spule als integraler Baustein der Platine geschützter ist als auch die Bauteile innerhalb der Kavitäten besser geschützt sind.

Typischerweise werden für Ausführungsformen weniger Montageschritte als bei bekannten Empfängern aus dem Stand der Technik benötigt oder die Montage kann kostengünstiger erfolgen.

### Kurze Beschreibung der Zeichnungen

Weitere Vorteile und Merkmale bevorzugter Ausführungsformen der Erfindung werden nachfolgend anhand der beiliegenden Zeichnungen erläutert, wobei die Figuren zeigen:
- Fig. 1: ist eine schematische Schnittansicht einer Ausführungsform der Erfindung;
- Fig. 2: zeigt eine weitere Ausführungsform in einer perspektivischen, schematischen Ansicht; und
- Fig. 3: zeigt den Ablauf eines erfindungsgemäßen Verfahrens.

### Beschreibung bevorzugter Ausführungsbeispiele

Nachfolgend werden typische Ausführungsformen anhand der Figuren beschrieben, wobei die Erfindung nicht auf die Ausführungsbeispiele beschränkt ist, vielmehr wird der Umfang der Erfindung durch die Ansprüche bestimmt.

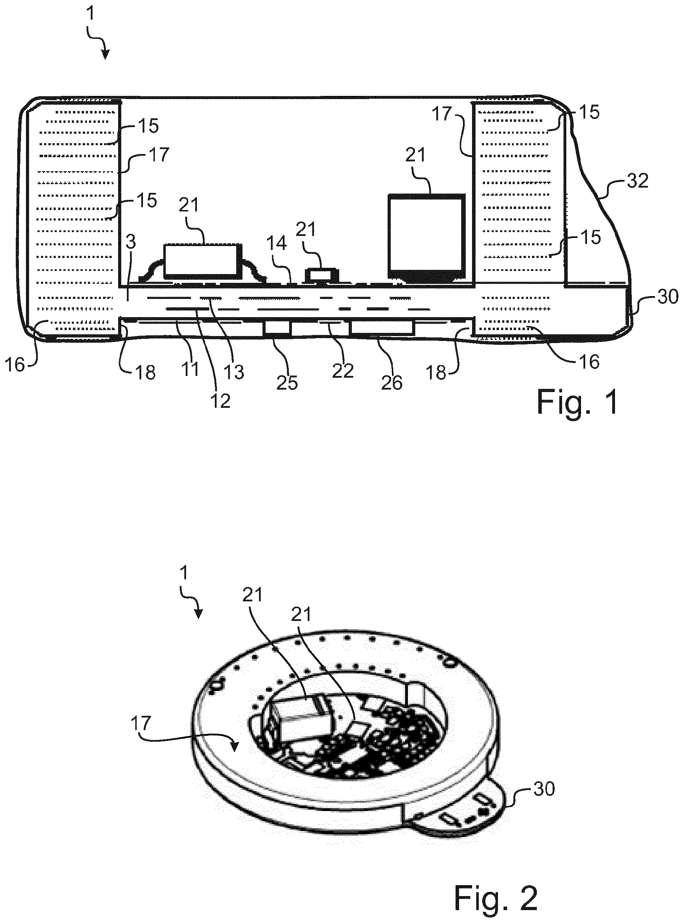

In der Fig. 1 ist ein Empfänger 1 gezeigt, welcher Teil eines Implantat-Systems sein kann, welches ein an den Empfänger 1 anschließbares elektromechanisches Implantat (nicht in den Figuren gezeigt) umfassen kann.

Der Empfänger 1 ist implantierbar und zur Übertragung von Energie zu dem Implantat geeignet. Hierzu umfasst der Empfänger 1 eine Spule, welche dazu geeignet ist, Energie zu übertragen oder zu empfangen, welche ausreichend ist, um einen elektromechanischen Antrieb eines Implantats, bspw. eines Marknagels, wie er beispielhaft in der DE 10 2011 053 638 A1 gezeigt ist, oder einer Skoliosebehandlungseinheit, wie sie beispielhaft in der DE 10 2010 047 738 A1 gezeigt ist, mit Energie zu versorgen.

Der Empfänger 1 ist geeignet, eine Dauerleistung von mindestens 1W an ein aktives oder mechatronisches Implantat zu übertragen.

Der Empfänger 1 umfasst eine mehrlagige Platine, welche eine Grundmembran 3 umfasst, die sich horizontal über den vollständigen Querschnitt der Platine ausdehnt. Die Grundmembran umfasst vier Lagen 11- 14, welche als elektrisch leitende, strukturierte Kupfer-Lagen ausgebildet sind.

In einem äußeren Spulenbereich bildet die Platine mit 15 Stück oberen weiteren Lagen 15 ein oberes Bord 17 und mit 3 Stück unteren weiteren Lagen 16 ein unteres Bord 18 aus. Nicht alle der weiteren Lagen sind aus Gründen der Übersichtlichkeit mit einem Bezugszeichen versehen.

Die Borde 17 und 18 sind jeweils umlaufend um einen Innenbereich, in welchem lediglich die vier Lagen 11 -14 der Grundmembran vorhanden sind.

Zwischen allen Lagen 11- 16 sind jeweils Prepregs als Isolationsschichten angeordnet, wobei die äußersten Lagen 11 und 14 im Innenbereich frei liegen, um auf einer Oberseite der Grundmembran (Lage 14) und auf einer Unterseite der Grundmembran (Lage 11) elektronische Bauelemente, welche auf der Oberseite beispielhaft mit den Bezugszeichen 21 gezeigt sind, oder Kontaktierungen 22 anzuordnen.

Im Spulenbereich bilden die Lagen 11 - 16, also sowohl die Lagen 11-14 der Grundmembran als auch die oberen und unteren weiteren Lagen 15 und 16, Spulenwindungen der Spule aus. Auf jeder Lage 11- 16 sind jeweils sieben (lediglich schematisch in Fig. 1 dargestellt) Windungen angeordnet. Auf diese Weise ist die Spule, integral in den Lagen der Platine im Spulenbereich aufgenommen.

Im Innenbereich können bei Ausführungsformen embedded parts in die Grundmembran integriert sein. Weiterhin können dort bspw. gehäuste oder ungehäuste Bauteile, ICs, Transistoren oder Widerstände angeordnet sein. Insbesondere auf der Unterseite oder auf der Oberseite können Testpunkte vorgesehen sein, welche ein Testen des Empfängers vor einem Umspritzen mit Silikon vereinfachen können. Weiterhin können Empfänger von Ausführungsformen eine Ferrithülse um den Spulenbereich oder eine Paste vorsehen, um den Wirkungsgrad der Spule zu verbessern.

Die Platine umfasst bei typischen Ausführungsformen aus dem Stand der Technik bekannte Leiterplattenmaterialen wie bspw. FR4 oder Polyimid.

Innerhalb des oberen Bords 17 wird eine obere Kavität gebildet. Innerhalb des unteren Bords 18 wird eine untere Kavität gebildet. Die elektronischen Bauelemente 21 der gezeigten Ausführungsform überragen den oberen Bord 17 nicht. Das bedeutet, dass die elektronischen Bauelemente 21 eine geringere Bauhöhe haben als die Höhe des Randes des Bords 17 über der Oberfläche der Oberseite der Grundmembran.

Die Höhe des Bordes kann bei Ausführungsformen als die Höhe zwischen der frei liegenden Lage oder obersten bzw. untersten Lage des Innenbereichs und dem Rand des Bordes definiert werden.

Ein in der unteren Kavität angeordneter Piezo-Summer 26 überragt den unteren Bord 18 lediglich um 10% der Höhe des unteren Bordes über der frei liegenden Lage 11 der Unterseite der Grundmembran. Auf diese Weise wird ein kompakter Aufbau geschaffen.

An einer Seite ist eine Nase 30 vorgesehen, welche aus den Lagen 11 - 14 der Grundmembran und der unteren weiteren16 gebildet wird. Die Nase 30 kann mit der frei liegenden Lage 14 für Kontaktierungen, beispielsweise zum Anschluss des Implantats, verwendet werden.

Die Kavitäten, welchen von den Borden 17 und 18 gebildet werden, sind mit Harz-Härter-Gemisch ausgegossen. Der gesamte Empfänger 1 ist mit Silikon 32 umspritzt, so dass er biokompatibel ist.

Die Fig. 2 zeigt eine weitere Ausführungsform eines Empfängers 1 zur Versorgung eines mechatronischen Implantats. Die Kavitäten des Empfängers 1 der Fig. 2 sind noch nicht mit Harz-Härter-Gemisch ausgegossen und es fehlt auch noch die Silikonschicht.

In der perspektivischen Darstellung der Figur 2 ist gut erkennbar, wie das obere Bord 17 einen Innenbereich umfängt, in welchem elektronische Bauelemente 21 geschützt angeordnet sein können.

Bei typischen Ausführungsformen sind auf der Oberseite oder der Unterseite oder auf beiden Seiten der Grundmembran im Innenbereich elektronische Bauelemente angeordnet. Die Anordnung bietet einen guten Schutz vor mechanischen Einflüssen.

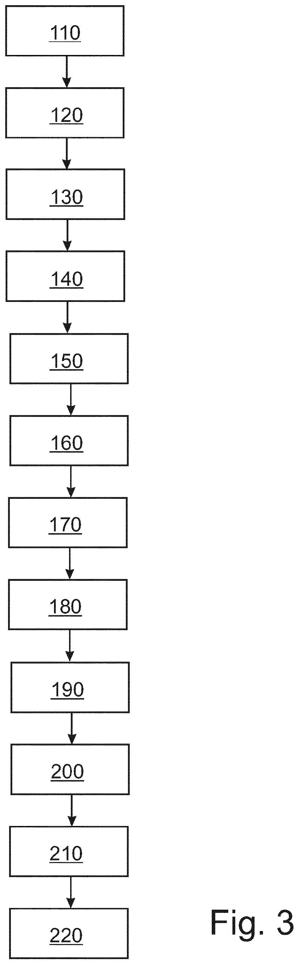

Die Fig. 3 zeigt ein typisches Verfahren zur Herstellung eines Empfängers. Das Verfahren startet in Block 110, wobei eine 4-lagige Grundmembran hergestellt wird. Dabei werden abwechselnd strukturierte Lagen aus Kupfer (Cores) und Isolationsschichten (Prepregs) aufgebracht und verpresst.

Die Grundmembran besteht aus vier Kupferschichten (die Lagen) und drei Isolationsschichten, welche strukturiert, gebohrt und galvanisiert werden. Die obere Kupferschicht bildet im fertigen Empfänger die Lage an der Oberseite der Grundmembran in der oberen Kavität, auf welcher die Bauteile bestückt werden. Auf der untersten Lage der Grundmembran befinden sich im fertigen Produkt die Testpunkte.

In einem Block 120 wird auf der Grundmembran an der Oberseite und der Unterseite ein im Innenbereich, d.h. im Bereich der Kavitäten ausgefrästes Prepreg aufgelegt.

In einem Block 130 werden in diese Ausfräsungen Teflonscheiben eingelegt. Diese Scheiben verbinden sich im Gegensatz zum Isolationsmaterial nicht mit der Kupferschicht der äußersten Lagen der Grundmembran.

In einem Block 140 werden weitere Lagen und Prepregs als Isolationsmaterial auf der Oberseite und auf der Unterseite der Grundmembran aufgebracht. Die jeweils äußerste Schicht ist Isolationsmaterial.

In einem Block 150 wird in die so entstandene Rohplatine mit jeweils einer Tiefenfräsung bis in die Tiefe der Teflonscheibe eine Kontur gefräst. Dadurch entsteht jeweils eine Kavität an der Oberseite und der Unterseite in der Rohplatine.

In einem Block 160 werden die Teflonscheiben aus der Rohplatine entnommen.

In einem Block 170 werden die Oberflächen der Kavitäten mit einer dünnen Schicht Nickel (bspw. 3-10 nm) und einer dünneren Schicht Gold (ca. 0,5-3 nm) veredelt.

In einem Block 180 wird auf die veredelten Oberflächen der Kavitäten eine Lotpaste appliziert. z.B. durch Jetprinten oder Dispensen.

In einem Block 190 werden die Footprints, das sind für Bauteile vorgesehene Landeflächen, in den Kavitäten mit elektronischen Bauelementen bestückt.

In einem Block 200 schmilzt im Lötofen, typischerweise mit Dampfphase, um eine gleichmäßige Wärmeverteilung zu erreichen, das Lötzinn unter den Füßchen der elektronischen Bauelemente, wobei sich die Bauteile mit der Leiterplatte verbinden.

In einem Block 210 werden die Kavitäten mit einem Harz-Härter-Gemisch ausgegossen, bis die Kavitäten zumindest im Wesentlichen eben ausgefüllt sind. Hierfür wird typischerweise keine Vergussform verwendet. Typischerweise erfolgt das Vergießen Vergussform-los.

In einem Block 220 wird die so entstandene Platine mit Silikon umspritzt, um den Empfänger mit der Platine biokompatibel zu machen. Damit ist das in der Fig. 3 gezeigte Verfahren beendet.

## Patentansprüche

1. Empfänger (1), insbesondere implantierbarer Empfänger (1) zur Übertragung von Energie zu einem Implantat, mit
- einer mehrlagigen Platine, welche eine Mehrzahl von elektrisch leitenden Lagen (11 - 16) umfasst,
- wobei die Platine einen äußeren Spulenbereich und einen von dem Spulenbereich umfangenen mehrlagigen Innenbereich umfasst,
- einer Spule, welche zumindest teilweise in den Lagen (11 - 16) der Platine im Spulenbereich integral aufgenommen ist,
- wobei innerhalb dieses Innenbereichs die Anzahl der Lagen (11 - 16) der Platine geringer ist als in dem Spulenbereich.

2. Empfänger (1) nach Anspruch 1, wobei der Innenbereich höchstens halb so viele Lagen umfasst wie der Spulenbereich.

3. Empfänger (1) nach einem der vorhergehenden Ansprüche, wobei der Spulenbereich auf einer Oberseite der Platine ein oberes Bord (17) um den Innenbereich ausbildet und wobei in dem Innenbereich auf der Oberseite der Platine elektronische Bauelemente (21) angeordnet sind.

4. Empfänger (1) nach einem der vorhergehenden Ansprüche, wobei auf einer Unterseite der Platine der Spulenbereich ein unteres Bord (18) ausbildet, welches auf der Unterseite eine untere Kavität im Innenbereich umfängt.

5. Empfänger (1) nach einem der vorhergehenden Ansprüche, wobei die Platine einstückig ausgeführt ist.

6. Empfänger (1) nach einem der vorhergehenden Ansprüche, mit einer funkwellenlosen Rückmeldeeinrichtung (26), welche dazu eingerichtet ist, eine Rückmeldung über einen Betriebszustand eines an den Empfänger (1) angeschlossenen Implantats zu erzeugen.

7. Empfänger (1) nach einem der vorhergehenden Ansprüche, wobei die Kavität oder die Kavitäten ausgegossen sind und/oder eine Umspritzung um den Empfänger (1) mit einem biokompatiblen Material vorgesehen ist.

8. Implantierbares System mit einem Empfänger (1) nach einem der vorhergehenden Ansprüche und einem elektromechanischen Implantat.

9. Verfahren zum Herstellen eines Empfängers (1), insbesondere implantierbarer Empfänger (1) zur Übertragung von Energie zu einem Implantat, welcher eine Platine umfasst, mit:
- Herstellen einer mehrlagigen Grundmembran der Platine mit einer Mehrzahl von Lagen (11- 14);
- Aufbau von weiteren Lagen (15, 16) auf einer Oberseite und/oder einer Unterseite der Grundmembran;
- wobei zumindest in einem Teil der weiteren Lagen (15, 16) im Spulenbereich Windungen einer Spule integriert sind; und
- Schaffen einer oberen Kavität oder einer unteren Kavität im Innenbereich innerhalb des Spulenbereichs durch Abtragen der weiteren Lagen (15, 16) im Innenbereich.

10. Verfahren nach Anspruch 9, wobei der Innenbereich der Grundmembran auf zumindest einer der Oberseite und der Unterseite während des Aufbauens der weiteren Lagen (15, 16) durch eine Schutzschicht bedeckt wird.

11. Verfahren nach Anspruch 9 oder 10 weiterhin umfassend:
- Bestücken des Innenbereichs an der Oberseite mit elektronischen Bauelementen (21),
- Ausgießen der oberen Kavität mit einem Harz-Härter-Gemisch, und/oder
- Umspritzen des Empfängers (1) mit einem biokompatiblen Material.

## Claims

1. Receiver (1), in particular an implantable receiver (1) for transmitting energy to an implant, with
- a multi-layer circuit board comprising a plurality of electrically conductive layers (11-16),
- wherein the circuit board comprises an outer coil area and a multi-layer inner area enclosed by the coil area,
- a coil which is integrally received at least partially in the layers (11-16) of the circuit board in the coil area,
- wherein the number of the layers (11-16) of the circuit board is smaller within this inner area than in the coil area.

2. Receiver (1) according to Claim 1, wherein the inner area comprises at most half as many layers as the coil area.

3. Receiver (1) according to either of the preceding claims, wherein the coil area, on an upper side of the circuit board, forms an upper rim (17) around the inner area, and wherein electronic components (21) are arranged on the upper side of the circuit board in the inner area.

4. Receiver (1) according to one of the preceding claims, wherein the coil area forms a lower rim (18) on an underside of the circuit board, which lower rim (18) on the underside encircles a lower cavity in the inner area.

5. Receiver (1) according to one of the preceding claims, wherein the circuit board is formed in one piece.

6. Receiver (1) according to one of the preceding claims, with a feedback device (26) which works without radio waves and which is designed to generate feedback concerning an operating state of an implant attached to the receiver (1).

7. Receiver (1) according to one of the preceding claims, wherein the cavity or the cavities are filled and/or the receiver (1) is encapsulated with a biocompatible material.

8. Implantable system with a receiver (1) according to one of the preceding claims and with an electromechanical implant.

9. Method for producing a receiver (1), in particular an implantable receiver (1) for transmitting energy to an implant and comprising a circuit board, by:
- producing a multi-layer base membrane of the circuit board with a plurality of layers (11-14);
- building up further layers (15, 16) on an upper side and/or an underside of the base membrane;
- wherein turns of a coil are integrated at least in some of the further layers (15, 16) in the coil area; and
- creating an upper cavity or a lower cavity in the inner area to the inside of the coil area, by removing the further layers (15, 16) in the inner area.

10. Method according to Claim 9, wherein the inner area of the base membrane is covered, on at least one of upper side and underside, by a protective layer during the building up of the further layers (15, 16).

11. Method according to Claim 9 or 10, further comprising:
- populating the inner area with electronic components (21) on the upper side,
- filling the upper cavity with a resin-catalyst mix, and/or
- encapsulating the receiver (1) with a biocompatible material.

## Revendications

1. Récepteur (1), notamment récepteur implantable (1) pour la transmission d'énergie à un implant, avec
- une plaquette multicouche comportant une pluralité de couches électriquement conductrices (11-16),
- dans lequel la plaquette comporte une zone de bobine extérieure et une zone intérieure multicouche entourée par la zone de bobine,
- une bobine qui est au moins partiellement reçue intégralement dans les couches (11-16) de la plaquette dans la zone de bobine,
- dans lequel, dans cette zone intérieure, le nombre de couches (11-16) de la plaquette est inférieur à celui dans la zone de la bobine.

2. Récepteur (1) selon la revendication 1, dans lequel la zone intérieure comporte tout au plus la moitié du nombre de couches que la zone de bobine.

3. Récepteur (1) selon l'une des revendications précédentes, dans lequel la zone de bobine forme, sur une face supérieure de la plaquette, un bord supérieur (17) autour de la zone intérieure et dans lequel sont disposés, dans la zone intérieure, sur la face supérieure de la plaquette, des composants électroniques (21).

4. Récepteur (1) selon l'une des revendications précédentes, dans lequel, sur une face inférieure de la plaquette, la zone de bobine forme un bord inférieur (18) qui entoure, sur la face inférieure, une cavité inférieure dans la zone intérieure.

5. Récepteur (1) selon l'une des revendications précédentes, dans lequel la plaquette est réalisée d'une seule pièce.

6. Récepteur (1) selon l'une des revendications précédentes, avec un dispositif de rétroaction sans ondes radioélectriques (26) qui est conçu pour générer une rétroaction sur un état de fonctionnement d'un implant raccordé au récepteur (1).

7. Récepteur (1) selon l'une des revendications précédentes, dans lequel la ou les cavités sont remplies et/ou il est prévu un surmoulage d'un matériau biocompatible autour du récepteur (1).

8. Système implantable avec un récepteur (1) selon l'une des revendications précédentes et un implant électromécanique.

9. Procédé de fabrication d'un récepteur (1), en particulier d'un récepteur implantable (1) pour la transmission d'énergie à un implant qui comporte une plaquette, avec le fait de :
- fabriquer une membrane de base multicouche de la plaquette avec une pluralité de couches (11-14) ;
- montage d'autres couches (15, 16) sur une face supérieure et/ou une face inférieure de la membrane de base ;
- dans lequel les enroulements d'une bobine sont intégrés dans la zone de la bobine au moins dans une partie des autres couches (15, 16) ; et
- créer une cavité supérieure ou une cavité inférieure dans la zone intérieure dans la zone de la bobine en éliminant les autres couches (15, 16) dans la zone intérieure.

10. Procédé selon la revendication 9, dans lequel la zone intérieure de la membrane de base est recouverte par une couche de protection sur au moins l'une parmi la face supérieure et la face inférieure pendant le montage des autres couches (15, 16).

11. Procédé selon la revendication 9 ou 10, comportant par ailleurs le fait de :
- équiper la zone intérieure sur la face supérieure de composants électroniques (21),
- verser dans la cavité supérieure un mélange de résine et d'un agent durcissant, et/ ou
- injecter autour du récepteur (1) un matériau biocompatible.
